# EUROPEAN PATENT SPECIFICATION

(11) **EP 1 620 393 B1**
(45) Date of publication and mention of the grant of the patent: **04.11.2009**
(21) Application number: 04726679.6
(22) Date of filing: 08.04.2004
(51) Int. Cl.: C07C 273/02, C07C 275/02

(54) **PROCESS FOR THE PREPARATION OF A UREA AND FORMALDEHYDE-CONTAINING AQUEOUS SOLUTION**
VERFAHREN ZUR HERSTELLUNG EINER HARNSTOFF UND FORMALDEHYD ENTHALTENDEN WÄSSRIGEN LÖSUNG
PROCEDE DE PREPARATION D'UNE SOLUTION AQUEUSE A BASE D'UREE ET DE FORMALDEHYDE

(30) Priority: 29.04.2003 NL 1023303
(43) Date of publication of application: 01.02.2006
(73) Proprietor: Prospect Management BV, 6343 CC Klimmen (NL)
(72) Inventor: VERSCHOOR, Hendrinus, Marinus, 6343 CC Klimmen (NL)
(74) Representative: Dohmen, Johannes Maria Gerardus
(86) International application number: PCT/NL2004/000235
(87) International publication number: WO 2004/096758

(56) References cited:
- WO-A-02/090323
- DE-B- 1 239 290
- DE-B- 1 593 053
- FR-A- 1 542 109
- US-A- 4 231 961

## Description

The invention relates to a process for the preparation of a urea and formaldehyde-containing aqueous solution (UFC),wherein the molar ratio between urea and formaldehyde (U/F) in the solution is between 0.5 and 0.001. UFC's, also known as urea-formaldehyde precondensates, are nowadays primarily used as additive to a urea melt before granulation or prilling to improve the properties of the urea granules. UFC is also used, but in a minor amount, as a starting material for the production of urea formaldehyde resins.

FR 1.542.109 relates to urea formaldehyde concentrates of aqueous solutions containing about 80 to 90% by weight partially reacted urea and formaldehyde in a molar ratio above 1:1 but less than 2:1 and are prepared by a process comprising preparing an aqueous mixture of urea and formaldehyde having a specific urea-formaldehyde mole ratio, adding ammonia, heating said mixture and boiling while maintaining the pH of the mixture in a range of about 8.5 to 10.

German Auslegeschrift 1 239 290 relates to a process for producing concentrated urea-formaldehyde solutions by absorbing gaseous formaldehyde in aqueous urea solutions.

According to the state of the art urea formaldehyde resins are produced by using urea granules and an aqueous formaldehyde solution as starting materials. These starting materials often are transported over large distances to the plant for production of the urea formaldehyde resins. The transport of the formaldehyde solution involves environmental and safety hazards. Therefore, a plant for production of the urea formaldehyde resins is often located in the vicinity of a formaldehyde plant. To produce a urea formaldehyde resin the urea granules are dissolved in water and/or in an aqueous formaldehyde solution.

A disadvantage from the process according to the state of the art is that the urea granules have to be prepared and transported over large distances and thereafter the urea has to be dissolved in water again to be able to prepare the resin.

The invention alms to eliminate the aforementioned disadvantage.

This aim is achieved by the preparation of a UFC by combining a formaldehyde-containing stream with a urea-containing aqueous stream that is supplied from a urea plant.

By preparing the UFC in such a way the water in the urea-containing aqueous stream does not have to be evaporated to be able to form granules or prills from the urea stream. Also the formation of prills or granules is no longer necessary. In this way a lot of energy, investments and labour costs, used for the evaporation of the water and the formation of prills or granules, can be saved.

An other advantage of the process according to the invention is that the UFC that is prepared is stable for a long period of time and can, without substantial any environmental or safety hazards be transported over large distances.

A further advantage is that, when prepared in this way, it is economically feasible to produce large quantities of UFC, so that UFC becomes available as a raw material for the production of UF-resins.

A UFC is a urea-formaldehyde precondensate, which is an aqueous solution containing urea and formaldehyde in a free and/or dissolved and/or reacted form. A UFC has a low viscosity which makes it suitable for storage in tanks and transportation by pumps. A UFC contains a molar excess of formaldehyde relative to the amount of urea, so that the UFC remains stable for a long period and is suitable for transportation over long distances. The UFC may contain traces of other compounds. Examples of such compounds are unreacted starting products or by-products from the synthesis of formaldehyde and/or urea, such as, for instance, methanol, CO₂, NH₃.

Since urea and formaldehyde are highly water-soluble, and since the mutual molar ratio between urea and formaldehyde (U/F) in the UFC may vary between a molar ratio of 0.5 and 0.001, the mutual ratios between water, formaldehyde and urea in the UFC may vary as well.

The quantity of water in the UFC can vary between 5 wt% and 50 wt%, more preferably between 10 wt% and 40 wt%, still more preferably between 15 wt% and 30 wt%.

The formaldehyde-containing stream comprises formaldehyde. Formaldehyde in the framework of the invention refers to the compound itself or compounds from which formaldehyde may be released, such as paraformaldehyde. Paraformaldehyde is an oligomeric form of formaldehyde.

The formaldehyde-containing stream is gaseous The formaldehyde-containing stream preferably consist primarily of formaldehyde, as in the case of a gaseous stream of formaldehyde originating from a formaldehyde plant. The formaldehyde-containing stream may also contain traces of other compounds. Examples of such compounds are unreacted starting products or by-products from the synthesis of formaldehyde such as, for instance, methanol, but also stabilisers.

The formaldehyde-containing stream is gaseous and, in a preferred embodiment of the invention, originates from a formaldehyde plant wherein formaldehyde is prepared by a process known per se; this is commonly done in a catalyzed reaction at high temperature with methanol as a feedstock. The advantage of this is that the formaldehyde-containing stream may be fed directly from the formaldehyde plant to the combination step according to the invention.

The urea-containing aqueous stream contains urea and water. The urea content in the stream may vary and lies between wide limits, usually between 50 and 95 wt%. The quantity of water in the urea-containing aqueous stream is usually between 50 wt% and 5 wt%. The urea-containing aqueous stream may also contain traces of other compounds. Examples of such compounds are unreacted starting products or by-products from the synthesis of urea, such as, for instance, ammoniumcarbamate, CO₂, NH₃.
In the light of the generally unwanted interaction between NH₃ and formaldehyde it is preferable to limit the weight quantity of NH₃ in the urea-containing aqueous stream such that the weight quantity of NH₃ in the urea-containing aqueous stream is less than 5%, preferably less than 1%.

The urea-containing aqueous stream is supplied from a urea synthesis plant. A urea synthesis plant normally comprises a urea synthesis section and on or more recovery sections, followed by an evaporation and finishing section. Examples of finishing sections are prilling or granulation sections. A urea synthesis section refers to a section in which NH₃ and CO₂ or compounds thereof such as ammonium carbamate, are reacted to form urea. Water is released in this equilibrium reaction. The stream that leaves the synthesis section is a synthesis solution; this is an aqueous solution which contains, besides urea, other compounds such as non-reacted starting materials. If, as has been customary until now, the intention is to obtain urea in a virtually pure form, the synthesis solution undergoes a number of follow-up operations in one or more recovery sections. Examples of such follow-up operations known per se are decomposition and separation steps such as stripping, whether or not in combination with pressure reduction and with the aim of removing unwanted starting products and by-products, and concentration steps with the aim of reducing the water content. In the framework of the present invention the synthesis solution and/or one or more urea-containing aqueous streams from the recovery section may serve as a urea-containing aqueous stream so long as the water content is higher than 5 wt% and preferably higher than 10 wt%. Thus, there are various possibilities of removing a urea-containing aqueous stream from the urea plant: either directly (from the synthesis section) or from or after one or more of the recovery sections. Normally the water content in the urea-containing aqueous stream is too low after it has passed the evaporation section of a urea plant.

It is possible to combine one or more streams that are removed at various places from the urea plant after the synthesis section to the urea-containing aqueous stream.

It is possible to feed the whole quantity of urea which leaves the urea synthesis section and/or recovery section(s) in the form of the urea-containing aqueous stream to the combination step according to the invention. It is also possible to feed a proportion of the quantity of urea which leaves the urea synthesis section and/or recovery section(s) in the form of the urea-containing aqueous stream to the combination step according to the invention.

Preferably the urea-containing aqueous stream is more than 5 wt% of the total stream which leaves the urea synthesis section; more preferably more than 10 wt% or 15 wt%, still more preferably more than 20 wt%. Preferably the urea-containing aqueous stream is less than 95 wt% of the total stream which leaves the urea synthesis section; more preferably less than 75 wt%, still more preferably less than 50 wt%.

Given that at least a proportion of the synthesis solution does not undergo all follow-up operations for obtaining virtually pure urea, the process according to the invention offers a possibility of debottlenecking a urea plant without the capacity of all components, for instance the evaporation and finishing section, needing to be increased. The invention therefore also relates to a process for debottlenecking a urea plant wherein the urea plant comprises a synthesis section, one or more recovery sections, an evaporation section and a finishing section, wherein the capacity of the urea synthesis section is increased comparatively more than that of at least one of the recovery sections and/or of the evaporation and finishing section. Capacity of a section here refers to the maximum possible throughput (measured in volume or mass) in that section.

The process of the invention may be carried out in a plant for the preparation of a UFC as defined in claim 10.

In the combination step, the formaldehyde-containing stream and the urea-containing aqueous stream are contacted with each other so that the UFC is formed. In that process there occurs to a greater or lesser degree a reaction, usually an condensation reaction, between formaldehyde and urea. The said reaction generally proceeds spontaneously. Thus, many processes and conditions are suitable for implementation of the combination step. A special catalyst is not generally necessary. The combination step may be carried out both batch-wise and continuously. The combination step may be carried out at atmospheric pressure but if desired also at increased or reduced pressure. Usually atmospheric pressure will be chosen for reasons of economy. The temperature during the combination step may vary between wide limits and usually is between 50°C and 150°C, preferably the temperature is between 60°C and 100°C, more preferably between 70°C and 90°C.

The combination step may for example be carried out by passing the formaldehyde-containing stream through the urea-containing aqueous stream in for example a vessel or an absorption column. Such an absorption column is used in known processes for the preparation of formaldehyde so as to prepare formalin from a gaseous formaldehyde stream with the aid of water. It is possible in one embodiment of the invention to carry out the combination step in a process for the preparation of formaldehyde which comprises an absorption column, by feeding the urea-containing aqueous stream from the urea synthesis section instead of water to the absorption column.

If the total quantity of water in the urea-containing aqueous stream and the formaldehyde-containing stream is inadequate for the desired application, it is advantageous to supply water to the combination step; it is also possible to add water to the UFC already formed.

The thus formed UFC may be applied in many different ways. For example in urea formaldehyde (UF) resins or in melamine urea formaldehyde (MUF) resins. With these applications, if a molar excess of formaldehyde is present in the UFC, it may be necessary to supply urea to the UFC. As a result the formaldehyde:ureum ratio will drop so that in this way for example a UF resin may be prepared from a UFC. These steps are known to those skilled in the art.

The process according to the invention will be further elucidated by means of 2 figures and examples; without being limited thereto.

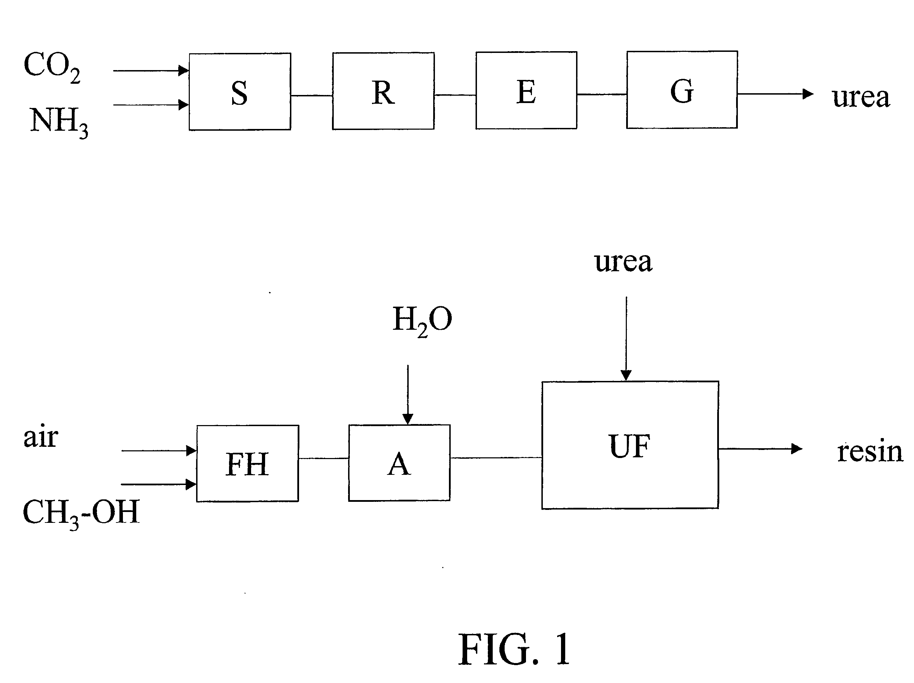

Figure 1 describes a process for the production of a UF resin according to the state of the art. Urea is produced in a urea plant on an other location than the location where the UF resin is produced. Urea is produced by adding CO₂ and NH₃ to the synthesis section (S) of a urea plant. The urea plant further comprises further a recovery section (R), an evaporation section (E) and a granulation section (G). The urea plant produces urea granules that can be stored and transported. On the same location as the location for the UF resin plant a formaldehyde plant is located, comprising a synthesis section (FH) and an absorption section (A). In the synthesis section air and methanol are introduced and formaldehyde is prepared at high temperature with the aid of a catalyst. The formaldehyde is then transported to an absorption section where the gaseous formaldehyde is absorbed in water. This formaldehyde solution is then added to a UF-resin plant. The urea needed for the preparation of the resin is supplied by transport over a large distance from the urea plant and the dry urea granules are first dissolved in water. This aqueous urea solution is then added to the resin plant for the preparation of the UF resin.

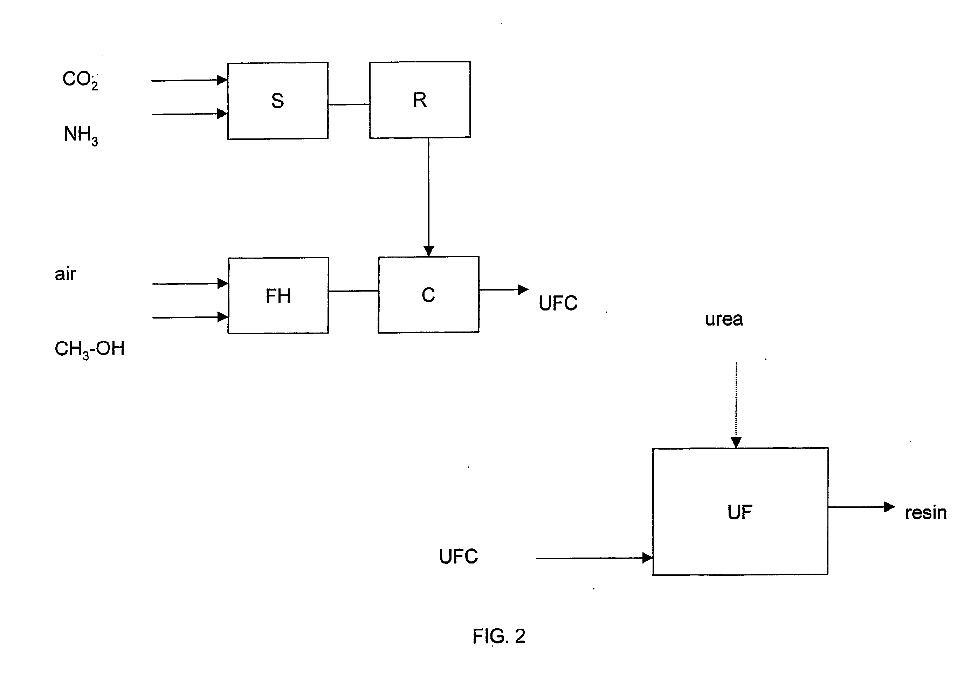

Figure 2 describes a process for the production of an UF resin (UF) with a UFC prepared according to the invention as a starting material. The resin plant is located on a location that differs from the location of the urea plant and the formaldehyde plant that are needed for the production of the UFC. The UFC is transported to the plant for the preparation of UF resin. To the resin plant, optionally, also urea in the form of granules or an aqueous urea solution, is added. Urea is produced by adding CO₂ and NH₃ to the synthesis section (S) of a urea plant. The urea plant further comprises further a recovery section (R) and thereafter the obtained urea-containing aqueous stream is added to the combination section according to the invention. In the formaldehyde synthesis section (FH) formaldehyde is is prepared at high temperature with the aid of a catalyst. The formaldehyde gas thus obtained is also added to the combination section according to the invention where it is absorbed in the urea-containing aqueous stream to prepare the UFC.

### Example I

A part of a urea-containing aqueous stream separated just before the evaporation section of a urea plant was added to the absorption column of a formaldehyde plant. The absorption column contained a formaldehyde-containing stream. The amount of formaldehyde-containing stream in relation to urea-containing aqeous stream was adjusted to 3:1. The urea-containing aqueous stream contained 77 wt% urea and the formaldehyde-containing stream contained 50 wt% formaldehyde. The stream leaving the absorption column was an UFC with a solid content of 56.8 wt% and a U/F molar ratio of 0.26.

### Example II; batch production

To prepare 1000 g of UF resin 779.7 g of the UFC of example I was added to a 2L reactor. The UFC contained 43.2 wt% water, 19,3 wt% urea and 37.5 wt% formaldehyde. The pH was adjusted to 8.0 with NaOH and 142.3 g of urea was added to obtain a U/F molar ratio of 0.5.

This solution was heated to 95 °C. After ten minutes at this temperature the pH was lowered to 5.0 with a solution of formic acid. When the pH reached 5.0 the condensation of the resin began and was continued untill a vicosity of about 300 mPa.s was reached.

Thereafter the pH of the solution was raised untill 8.0 with a NaOH solution and 194.9 g urea was added to obtain a U/F molar ratio of 0.83. The temperature was raised to 80°C and 116.9 g of water was evaporated to adjust the solid content of the resin. Thereafter the solution was cooled to room temperature. The resin thus obtained is suitable for use as a glue in the woodprocessing industry.

### Example III; continuous production

To prepare a resin continuously the UFC prepared according to Example I is dosed to a pipe reactor through two dosing points and there contacted with a urea-containing aqueous stream. The pipe reactor also contains dosing points for dosing NaOH and formic acid. Just after the entrance of the pipe reactor the UFC was brought to a temperature of 85 °C and a pH of 5.0. The ratio between the UFC stream and the urea-containing aqueous stream was adjusted in such a way that a U/F molar ratio was obtained of 0.5 in the first part of the reactor. The ratio between the UFC and the urea-containing aqueous stream was thus 1:0.24.

At 80% of the reactor the pH was adjusted to 8.0 whereafter the ratio between the UFC and the urea-containing aqueous stream was changed to 1:0.32.

At the end of the reactor a UF resin was obtained with a U/F molar ratio of 0.83 and a solid content of 70 wt.%. The resin thus obtained is suitable for use as a glue in the woodprocessing industry.

## Claims

1. The use of a urea-containing aqueous stream that is supplied from a urea plant for the preparation of a urea and formaldehyde-containing aqueous solution (UFC), wherein the molar ratio between urea and formaldehyde (U/F) in the solution is between 0.5 and 0.001, **characterised in that** the UFC is prepared by combining a gaseous formaldehyde-containing stream with said urea-containing stream.

2. The use according to claim 1 wherein the formaldehyde-containing stream is supplied from a formaldehyde plant.

3. The use according to claim 1 wherein the urea-containing aqueous stream contains between 5 wt% and 50 wt% of water.

4. The use according to claim 1 wherein the urea-containing aqueous stream contains less than 5 wt% of NH₃.

5. The use according to claim 1 wherein the urea-containing aqueous stream undergoes between the urea synthesis section and the combination step one or more decomposition and/or separation steps wherein the quantities of ammonium carbamate and/or NH₃ and/or CO₂ in the urea-containing aqueous stream are reduced.

6. The use according to claim 1 wherein the urea-containing aqueous stream undergoes between the urea synthesis section and the combination step one or more concentration steps wherein the quantity of H₂O in the stream is reduced to no less than 5 wt%.

7. The use according to claim 1, wherein the urea-containing aqueous stream is between 5 wt% and 95 wt% of the total stream that is released from the urea plant.

8. The use according to claim 1 wherein the combination step is carried out in a process for the preparation of formaldehyde, wherein in the process a formaldehyde-containing stream is prepared and wherein the process comprises an absorption column, **characterized in that** the urea-containing aqueous stream from the urea synthesis section and the formaldehyde-containing stream are supplied to an absorption column.

9. The use according to claim 1 wherein water is supplied to the combination step or to the UFC.

10. A plant for the preparation of a urea and formaldehyde-containing aqueous solution (UFC), comprising:
- a urea synthesis section wherein a urea-containing aqueous stream is formed;
- a formaldehyde synthesis section, and
- a combination section wherein the urea-containing aqueous stream is contacted with a formaldehyde-containing stream so that the UFC is formed, **characterized in that** the combination section is an absorption column wherein a gaseous formaldehyde-containing stream from the formaldehyde synthesis section is contacted with the urea containing aqueous stream, and optionally a recovery section located between said urea synthesis section and said combination section wherein compounds other than urea may be removed wholly or partially from the urea-containing aqueous stream, wherein at least a proportion of the urea-containing aqueous stream from the urea synthesis section and/or from the recovery section is supplied to the combination section.

## Patentansprüche

1. Verwendung eines harnsäureenthaltenden wässrigen Stroms, der von einer Harnsäureanlage geliefert wird für die Herstellung einer Harnsäure und Formaldehyd enthaltenden wässrigen Lösung (UFC), wobei das Molarverhältnis zwischen Harnsäure und Formaldehyd (U/F) in der Lösung zwischen 0,5 und 0,001 liegt, **dadurch gekennzeichnet, dass** die Harnsäure und Formaldehyd enthaltende wässrige Lösung (UFC) hergestellt wird durch Kombinieren eines gasförmigen Formaldehyd enthaltenden Stroms mit dem Harnsäure enthaltenden Strom.

2. Verwendung nach Anspruch 1, wobei der Formaldehyd enthaltende Strom von einer Formaldehydanlage geliefert wird.

3. Verwendung nach Anspruch 1, wobei der Harnsäure enthaltende wässrige Strom zwischen 5 Gewichts% und 50 Gewichts% Wasser enthält.

4. Verwendung nach Anspruch 1, wobei der Harnsäure enthaltende wässrige Strom weniger als 5 Gewichts% NH₃ enthält.

5. Verwendung nach Anspruch 1, wobei der Harnsäure enthaltende wässrige Strom zwischen dem Harnsäuresyntheseabschnitt und dem Kombinationsschritt einen oder mehrere Zersetzungs- und/oder Trennschritte durchläuft in denen die Mengen von Ammoniumcarbamat und/oder NH₃ und/oder CO₂, in dem Harnsäure enthaltenden wässrigen Strom reduziert werden.

6. Verwendung nach Anspruch 1, wobei der Harnsäure enthaltende wässrige Strom zwischen dem Harnsäuresyntheseabschnitt und dem Kombinationsschritt einen oder mehrere Konzentrationsschritte durchläuft, in denen die Menge von H₂O in dem Strom auf nicht weniger als 5 Gewichts% reduziert wird.

7. Verwendung nach Anspruch 1, wobei der Harnsäure enthaltende wässrige Strom zwischen 5 Gewichts% und 95 Gewichts% des Gesamtstroms ist, der aus der Harnsäureanlage freigegeben wird.

8. Verwendung nach Anspruch 1, wobei der Kombinationsschritt in einem Prozess für die Herstellung von Formaldehyd durchgeführt wird, wobei in dem Prozess ein Formaldehyd enthaltender Strom hergestellt wird, und wobei der Prozess eine Absorptionssäule aufweist, **dadurch gekennzeichnet, dass** der Harnsäure enthaltende wässrige Strom aus dem Harnsäuresyntheseabschnitt und der Formaldehyd enthaltende Strom an einer Absorptionssäule geliefert werden.

9. Verwendung nach Anspruch 1, wobei Wasser zu dem Kombinationsschritt oder zu der Harnsäure und Formaldehyd enthaltenden wässrigen Lösung (UFC) hinzugefügt wird.

10. Eine Anlage für die Herstellung einer Harnsäure und Formaldehyd enthaltenden wässrigen Lösung (UFC), die folgendes aufweist:
einen Harnsäuresyntheseabschnitt, in dem ein Harnsäure enthaltender wässriger Strom gebildet wird;
ein Formaldehydsyntheseabschnitt; und
ein Kombinationsabschnitt, in dem der Harnsäure enthaltende wässrige Strom mit einem Formaldehyd enthaltenden Strom kontaktiert wird, so dass die Harnsäure und Formaldehyd enthaltende wässrige Lösung (UFC) gebildet wird, **dadurch gekennzeichnet, dass** der Kombinationsabschnitt eine Absorptionssäule ist, in der ein gasförmiger Formaldehyd enthaltender Strom aus dem Formaldehydsyntheseabschnitt mit dem Harnsäure enthaltenden wässrigen Strom kontaktiert wird, und optional einem Wiedergewinnungsabschnitt, der zwischen dem Harnsäuresyntheseabschnitt und dem Kombinationsabschnitt angeordnet ist, wobei andere Verbindungen als Harnsäure vollständig oder teilweise aus dem Harnsäure enthaltenden wässrigen Strom entfernt werden, wobei wenigstens ein Teil des Harnsäure enthaltenden wässrigen Stroms aus dem Harnsäuresyntheseabschnitt und/oder aus dem Wiedergewinnungsabschnitt zu dem Kombinationsabschnitt geliefert wird.

## Revendications

1. Utilisation d'un courant aqueux contenant de l'urée qui est fourni par une installation d'urée pour la préparation d'une solution aqueuse contenant de l'urée et du formaldéhyde (UFC) où le rapport molaire entre l'urée et le formaldéhyde (U/F) dans la solution est entre 0,5 et 0,001, **caractérisée en ce que** l'UFC est préparée en combinant un courant contenant du formaldéhyde gazeux avec ledit courant contenant de l'urée.

2. Utilisation selon la revendication 1 où le courant contenant du formaldéhyde est fourni par une installation de formaldéhyde.

3. Utilisation selon la revendication 1 où le courant aqueux contenant de l'urée contient entre 5 % en masse et 50 % en masse d'eau.

4. Utilisation selon la revendication 1 où le courant aqueux contenant de l'urée contient moins de 5 % en masse de NH₃.

5. Utilisation selon la revendication 1 où le courant aqueux contenant de l'urée subit entre la section de synthèse de l'urée et l'étape de combinaison une ou plusieurs étapes de décomposition et/ou de séparation où les quantités de carbamate d'ammonium et/ou de NH₃ et/ou de CO₂ dans le courant aqueux contenant de l'urée sont réduites.

6. Utilisation selon la revendication 1 où le courant aqueux contenant de l'urée subit entre la section de synthèse de l'urée et l'étape de combinaison une ou plusieurs étapes de concentration où la quantité de H₂O dans le courant est réduite à pas moins de 5 % en masse.

7. Utilisation selon la revendication 1 où le courant aqueux contenant de l'urée est entre 5 % en masse et 95 % en masse du courant total qui est libéré par l'installation d'urée.

8. Utilisation selon la revendication 1 où l'étape de combinaison est conduite dans un procédé pour la préparation de formaldéhyde, où dans le procédé un courant contenant du formaldéhyde est préparé et où le procédé comprend une colonne d'absorption, **caractérisée en ce que** le courant aqueux contenant de l'urée provenant de la section de synthèse d'urée et le courant contenant du formaldéhyde sont fournis à une colonne d'absorption.

9. Utilisation selon la revendication 1 où de l'eau est fournie à l'étape de combinaison ou à l'UFC.

10. Installation pour la préparation d'une solution aqueuse contenant de l'urée et du formaldéhyde (UFC) comprenant :
- une section de synthèse d'urée où un courant aqueux contenant de l'urée est formé ;
- une section de synthèse de formaldéhyde, et
- une section de combinaison où le courant aqueux contenant de l'urée est mis en contact avec un courant contenant du formaldéhyde de sorte que l'UFC est formée, **caractérisée en ce que** la section de combinaison est une colonne d'absorption où un courant contenant du formaldéhyde gazeux provenant de la section de synthèse de formaldéhyde est mis en contact avec le courant aqueux contenant de l'urée, et éventuellement une section de récupération située entre ladite section de synthèse d'urée et ladite section de combinaison où les composés différents de l'urée peuvent être retirés totalement ou partiellement du courant aqueux contenant de l'urée, où au moins une proportion du courant aqueux contenant de l'urée provenant de la section de synthèse d'urée et/ou provenant de la section de récupération est fournie à la section de combinaison.
